# EUROPÄISCHE PATENTSCHRIFT

(11) **EP 2 721 162 B1**
(45) Veröffentlichungstag und Bekanntmachung des Hinweises auf die Patenterteilung: **08.08.2018**
(21) Anmeldenummer: 12728212.7
(22) Anmeldetag: 13.06.2012
(51) Int. Cl.: C12P 7/06, C12P 7/16, C12P 7/28

(54) **ABE FERMENTATIONSVERFAHREN UMFASSEND DIE PRODUKTABSORPTION DURCH ISOPHORON**
ABE FERMENTATION PROCESS INVOLVING PRODUCT ABSORPTION BY ISOPHORONE
PROCÉDÉ DE FERMENTATION ABE COMPRENANT L'ABSORPTION DU PRODUIT PAR ISOPHORONE

(30) Priorität: 17.06.2011 DE 102011077705
(43) Veröffentlichungstag der Anmeldung: 23.04.2014
(73) Patentinhaber: Evonik Degussa GmbH, 45128 Essen (DE)
(72) Erfinder: NITZ, Jörg-Joachim, 45257 Essen (DE); GRUND, Gerda, 48653 Coesfeld (DE); BECKER, Franz Ulrich, 63579 Freigericht-Horbach (DE); STIER, Patrick, 63762 Großostheim (DE)
(86) Internationale Anmeldenummer: PCT/EP2012/061141
(87) Internationale Veröffentlichungsnummer: WO 2012/171929

(56) Entgegenhaltungen:
- WO-A2-2009/079362
- US-A1- 2009 162 912
- COLOMBO, A. ET AL.: "Liquid-Liquid Equilibria of the Ternary Systems Water + Acetic acid + Ethyl Acetate and Water + Acetic Acid + Isophorone (3,5,5-Trimethyl-2-cyclohexen-1-one)", JOURNAL OF CHEMICAL AND ENGINEERING DATA, Bd. 44, 1999, Seiten 35-39, XP002685675,

## Beschreibung

### Gebiet der Erfindung

Gegenstand der Erfindung ist ein neuartiges Verfahren zur kostengünstigen Aufarbeitung der niedermolekularen Produkte (Molekulargewicht < 100 g/mol), Aceton, Butanol und Ethanol in einem Fermentationsprozess. Die Aufarbeitung gelingt mittels Gasstripping kombiniert mit einer nachgeschalteten Absorption mit Isophoron als Absorptionsmittel.

### Stand der Technik

Die Lösungsmittel Aceton, Ethanol und Butanol sind wichtige Plattformchemikalien der Chemischen Industrie mit stetig steigendem Bedarf. Insbesondere für die Herstellung von Aceton und Butanol werden zum heutigen Zeitpunkt meist petrochemische Verfahren angewendet, die letztlich auf dem "Cracken" von Erdöl beruhen und somit langfristig nicht zukunftsweisend sind.
Es besteht daher ein großes Interesse alternative Herstellverfahren auf der Basis zukunftsträchtiger Kohlenstoffquellen, wie bspw. aus nachwachsenden Rohstoffen oder sogar gasförmigen Kohlenstoffquellen wie z. B. Kohlenstoffdioxid (CO₂) oder Kohlenstoffmonoxid (CO) zu entwickeln. Fermentationsprozesse können eine Alternative zur Herstellung von Plattform-, Spezial-Chemikalien und auch Treibstoff bieten.
Das größte Problem bei den meisten fermentativen Herstellungsverfahren ist die Trennung des organischen Produktes von der Fermentationsbrühe, da diese oft hoch komplex ist und vor allen Dingen einen hohen Wassergehalt aufweist. Große Wassermengen sind aus prozesstechnischer Sicht unerwünscht, da diese im Verfahren viel Energie benötigen.
Die klassische Aceton-Butanol-Ethanol- (ABE) Fermentation mit Clostridien (z. B.: *Clostridium acetobutylicum*) ist beispielsweise ein mikrobielles Verfahren zur Herstellung niedermolekularer, organischer Verbindungen, wurde bereits im Jahre 1916 industriell genutzt und liefert die Produkte Aceton-Butanol-Ethanol im Verhältnis 3:6:1 in der Fermentationsbrühe.
Qureshi und Blaschek beschreiben in Renewable Energy, 22(4): 557-564 und Ezeji et al. in Appl Microbiol Biotechnol, 63(6):653-8 und US 2009/0162912 A1 ein ABE-Verfahren, in dem die niedermolekularen,
organischen Verbindungen per Gasstripping aus der Fermentationsbrühe ausgetragen werden und in Kühlfallen adsorbiert werden.
Dieses Verfahren hat den Nachteil eines hohen Energiebedarfs, da die Kühlung kontinuierlich aufrechterhalten werden muss. Ein weiterer Nachteil ist, dass in den Kühlfallen die Produkte nur unzureichend adsorbiert werden, ein beträchtlicher Anteil somit aus der Falle wieder ausgetragen und gegebenenfalls in den Fermenter zurückgeführt wird, wodurch es zu unnötiger Produktinhibition kommen kann.

WO2009/079362 A2 offenbart ein Verfahren umfassend die Verfahrensschritte:
A) Bereitstellen einer wässrigen Lösung umfassend Butanol produzierende Mikroorganismen,
B) Einleiten mindestens eines Gases oder Gasgemisches in die wässrige Lösung,
C) Ausleiten des Gasstromes durch eine Ether, Aldehyd, Ester, Keton oder Alkohol enthaltende Zusammensetzung und gegebenenfalls
D) Abtrennen des Butanols von der Ether, Aldehyd, Ester, Keton oder Alkohol enthaltenden Zusammensetzung. Colombo et al. offenbaren in J. Chem. Eng. Data, 1999, 44 (1), pp 35-39, die Verwendung von Isophoron zur Flüssig-Flüssig-Extraktion von Essigsäure aus einer wässrigen Lösung. Aufgabe der Erfindung war es, ein Verfahren bereitzustellen welches in der Lage ist, mit einfachen und energieschonenden Hilfsmitteln eine Aufarbeitung der niedermolekularen, organischen Verbindungen aus der wässrigen Fermentationsbrühe zu ermöglichen.

### Beschreibung der Erfindung

Überraschenderweise wurde gefunden, dass das im Folgenden beschriebene Verfahren umfassend eine Absorption der niedermolekularen, organischen Verbindungen ausgewählt aus der Gruppe umfassend Aceton, Butanol, Ethanol mit Hilfe von Isophoron die der Erfindung gestellte Aufgabe zu lösen vermag.
Bei Isophoron handelt es sich um das trimere Kondensationsprodukt des Acetons und um ein hochsiedendes Lösemittel, das u. a. auch in der Lack-, Druckfarben-, Klebstoff- und Pflanzenschutzmittel-Industrie Verwendung findet.

Gegenstand der vorliegenden Erfindung ist daher ein einfaches und kostengünstiges Aufarbeitungsverfahren für fermentative Produktionsprozesse, wobei die Produkte *in-situ* aus der Fermentationsbrühe entfernt werden. Die Aufarbeitung gelingt mittels Gasstripping kombiniert mit einer nachgeschalteten Absorption mit Isophoron und gegebenenfalls anschließender Trennung in die Reinkomponenten.

Ein Vorteil der vorliegenden Erfindung ist es, dass das erfindungsgemäße Aufarbeitungsverfahren sowohl auf anaerobe, als auch auf aerobe Fermentationen angewendet werden kann. Das Gasstripping kann also sowohl mit elementar sauerstoffhaltigen Gasen als Schleppgas (insbesondere bei Prozessen unter aeroben Bedingungen), als auch mit beliebig elementar sauerstofffreien Gasen (insbesondere bei Prozessen unter anaeroben Bedingungen) durchgeführt werden.

Ein weiterer Vorteil der vorliegenden Erfindung ist es, dass die ausgestrippten niedermolekularen, organischen Verbindungen, Aceton, Butanol und Ethanol, insbesondere Aceton (nahezu) quantitativ mittels Gaswäsche in Isophoron als Absorptionsmittel aufkonzentriert werden können.
Ein weiterer Vorteil der vorliegenden Erfindung ist es, dass sich diese niedermolekularen, organischen Verbindungen wieder sehr einfach vom Isophoron trennen lassen und somit in hoher Reinheit gewonnen werden können.
Noch ein Vorteil der vorliegenden Erfindung ist es, dass die zur Versorgung der Mikroorganismen eingesetzten Gase auch gleichzeitig die Funktion des Schleppgases übernehmen können, dieses gilt insbesondere wenn die eingesetzten Gase Anteile von Kohlendioxid, Kohlenmonoxid, Wasserstoff, Synthesegas oder beliebige Mischungen der zuvor genannten Gase besitzen.
Desweiteren hat die vorliegende Erfindung den Vorteil, dass niedermolekuare Produkte *in-situ* aus der Fermentationsbrühe entfernt werden können, und somit sich in der Fermenterbrühe keine hohen Produktkonzentrationen aufbauen können, die auf die vorhandenen Mikroorganismen toxisch wirken können.
Das erfindungsgemäße Verfahren zur Herstellung von niedermolekularen, organischen Verbindungen umfasst die Verfahrensschritte
A) Bereitstellen einer wässrigen Lösung umfassend niedermolekulare, organische Verbindungen produzierende Mikroorganismen,
B) Einleiten mindestens eines Gases oder Gasgemisches in die wässrige Lösung,
C) Ausleiten des Gasstromes durch eine Isophoron enthaltende Zusammensetzung und gegebenenfalls
D) Abtrennen der niedermolekularen, organischen Verbindungen von der Isophoron enthaltenden Zusammensetzung
dadurch gekennzeichnet,
dass die niedermolekulare, organische Verbindung ausgewählt ist aus der Gruppe umfassend Aceton, Butanol, Ethanol, insbesondere Aceton. Unter dem Begriff "niedermolekular" wird die Eigenschaft verstanden, ein Molekulargewicht <100 g/mol aufzuweisen.
Alle angegebenen Prozent (%) sind, wenn nicht anders angegeben, Massenprozent.
Wenn nicht anders angegeben, sind bezüglich der Umgebungsparameter wie Druck, Temperatur Standardbedingungen gemeint.

Bevorzugte niedermolekulare, organische Verbindungen weisen einen Dampfdruck von größer 3 hPa, bevorzugt größer 50 hPa, insbesondere größer 150 hPa, bei 20°C auf.

Die in dem erfindungsgemäßen Verfahren eingesetzten Mikroorganismen sind insbesondere Hefen und Bakterien. Insbesondere sind die Mikroorganismen ausgewählt aus Gattungen der Gruppe umfassend, bevorzugt bestehend aus, *Clostridium, Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Pichia, Candida, Hansenula* und *Saccharomyces.* Beispielhafte Arten dieser Gattungen sind *Escherichia coli, Alcaligenes eutrophus, Bacillus licheniformis, Paenibacillus macerans, Rhodococcus erythropolis, Pseudomonas putida, Enterococcus faecium, Enterococcus gallinarium, Enterococcus faecalis, Bacillus subtilis* und *Saccharomyces cerevisiae.*
In dem erfindungsgemäßen Verfahren wird bevorzugt ein Mikroorganismus, ausgewählt aus der Gruppe *Escherichia coli, Corynebacterium glutamicum, Clostridium spec., Clostridium aceticum, Acetobacterium woodii, Clostridium acetobutylicum, Clostridium beijerinckii, Yarrowia lipolytica, Saccharomyces spec., Saccharomyces cerevisiae* und *Pichia pastoris* eingesetzt.
Es ist erfindungsgemäß bevorzugt, dass es sich bei den Mikroorganismen um gentechnisch veränderte Mikroorganismen handelt. Insbesondere werden hier solche Mikroorganismen eingesetzt, die derart gentechnisch verändert wurden, als dass sie mehr niedermolekulare, organische Verbindungen zu produzieren vermögen, als ihr Wildtyp.
Solche gentechnisch veränderten Mikroorganismen sind für die Herstellung von Aceton als niedermolekulare, organische Verbindung in beispielsweise der EP2181195 beschrieben. Die in dieser Anmeldung offenbarten Zellen werden erfindungsgemäß bevorzugt in dem erfindungsgemäßen Verfahren zur Herstellung von Aceton eingesetzt. Solche Zellen sind insbesondere für Herstellungsverfahren unter aeroben Bedingungen geeignet.
Weitere solcher gentechnisch veränderten Mikroorganismen für die Herstellung von Aceton als niedermolekulare, organische Verbindung sind in der WO2010121849 beschrieben. Die in dieser Anmeldung offenbarten Zellen werden erfindungsgemäß bevorzugt in dem erfindungsgemäßen Verfahren zur Herstellung von Aceton eingesetzt, insbesondere bei Herstellungsverfahren unter anaeroben Bedingungen von Aceton.
Gentechnisch veränderte Mikroorganismen für die Herstellung von Butanol als niedermolekulare, organische Verbindung sind beispielsweise in der WO2008124523, der WO2008052973 und der WO2008052596 beschrieben. Die in dieser Anmeldung offenbarten Zellen werden erfindungsgemäß bevorzugt in dem erfindungsgemäßen Verfahren zur Herstellung von Butanol eingesetzt.
Gentechnisch veränderte Mikroorganismen für die Herstellung von Ethanol als niedermolekulare, organische Verbindung sind beispielsweise in der WO2011003893 und der WO2010130806 beschrieben. Die in dieser Anmeldung offenbarten Zellen werden erfindungsgemäß bevorzugt in dem erfindungsgemäßen Verfahren zur Herstellung von Ethanol eingesetzt.

In dem erfindungsgemäßen Verfahren kann in Verfahrensschritt B) jedes beliebige Gas oder Gasgemische eingesetzt werden, welches mit den niedermolekularen, organischen Verbindungen kein unerwünschten Nebenreaktionen eingeht oder toxisch für die eingesetzten Mikroorgansimen ist.
Bevorzugt enthält das Gas oder Gasgemisch mindestens eines ausgewählt aus der Gruppe umfassend, bevorzugt bestehend aus, Luft, Synthesegas, Stickstoff, Kohlenstoffdioxid, Kohlenstoffmonoxid, Wasserstoff, Sauerstoff und Methan.

Es ist erfindungsgemäß bevorzugt, als Gas oder Gasgemische in Verfahrensschritt B) ein elementaren Sauerstoff enthaltendes Gas oder Gasgemisch einzusetzen, wenn in Verfahrensschritt A) die Mikroorganismen die niedermolekularen, organischen Verbindungen unter aeroben Bedingungen produzieren. Unter dem Begriff "aerobe Bedingung" wird im Zusammenhang mit der vorliegenden Erfindung verstanden, dass ein Sauerstoffpartialdruck von > 0,01 bar vorliegt. In diesem Zusammenhang enthält das Gas oder Gasgemisch mindestens eines ausgewählt aus der Gruppe umfassend, bevorzugt bestehend aus Luft oder elemetarem Sauerstoff.

Es ist erfindungsgemäß bevorzugt, als Gas oder Gasgemische in Verfahrensschritt B) ein Gas oder Gasgemisch einzusetzen, welches frei von elementarem Sauerstoff ist, wenn in Verfahrensschritt A) die Mikroorganismen die niedermolekularen, organischen Verbindungen unter anaeroben Bedingungen produzieren. Unter dem Begriff "anaerobe Bedingung" wird im Zusammenhang mit der vorliegenden Erfindung verstanden, dass ein Sauerstoffpartialdruck von ≤ 0,01 bar vorliegt. In diesem Zusammenhang enthält das Gas oder Gasgemisch bevorzugt mindestens eines ausgewählt aus der Gruppe umfassend, bevorzugt bestehend aus, Synthesegas, Stickstoff, CO₂, CO, H₂ und Methan.
In einer weiteren Ausführungsform des erfindungsgemäßen Verfahrens handelt es sich bei den in Verfahrensschritt A) eingesetzten Mikroorganismen um solche, die die klassische ABE-Fermentation in Verfahrensschritt A) durchführen; somit sind in diesem Zusammenhang die niedermolekularen, organischen Verbindungen Aceton, Ethanol und Butanol, insbesondere Aceton. Solche Organismen, die klassisch bei der ABE-Fermentation eingesetzt werden können, sind *Clostridium acetobutylicum* und *Clostridium beijerinckii,* welche sich unter strikt anaeroben Bedingungen vermehren und Mono-, Di- und Polysaccharide umsetzen.
Um das in dieser Verfahrensvariante in Verfahrensschritt B) eingesetzte Gas oder Gasgemisch handelt es sich bevorzugt um mindestens eines ausgewählt aus der Gruppe umfassend, bevorzugt bestehend aus, Stickstoff, Luft, Synthesegas, Kohlenstoffdioxid, Kohlenstoffmonoxid, H₂, Methan.

In einer bevorzugten Ausführungsform des erfindungsgemäßen Verfahrens handelt es sich um die niedermolekulare, organische Verbindung Aceton, und als Mikroorganismen werden solche eingesetzt, die Aceton ausgehend von Acetyl-CoenzymA Aceton über die enzymatische Umsetzung von Acetyl-CoA zu Acetoacetyl-CoA, die enzymatische Umsetzung von Acetoacetyl-CoA zu Acetoacetat und CoA, wobei das CoenzymA nicht auf ein Akzeptor-Molekül übertragen wird, und das Decarboxylieren von Acetoacetat zu Aceton und CO₂ produzieren.

In einer weiteren bevorzugten Variante des erfindungsgemäßen Verfahrens handelt es sich bei den in Verfahrensschritt A) eingesetzten Mikroorganismen um acetogene Mikroorganismen, die Aceton als niedermolekulare, organische Verbindung aus mindestens einer Kohlenstoffquelle ausgewählt aus der Gruppe enthaltend Kohlendioxid und Kohlenmonoxid zu bilden vermögen und um das in Verfahrensschritt B) eingesetzte Gas oder Gasgemisch um mindestens eines ausgewählt aus der Gruppe umfassend, bevorzugt bestehend aus, Stickstoff, Synthesegas, Kohlenstoffdioxid, Kohlenstoffmonoxid, H₂. In dieser Verfahrensvariante bevorzugt eingesetzte Zellen sind in der WO 2010/121849 beschrieben.

In Verfahrensschritt C) werden die niedermolekularen, organischen Verbindungen mindestens teilweise durch Ausleiten des Gasstromes aus der wässrigen Lösung durch eine Isophoron enthaltende Zusammensetzung geleitet. Bevorzugt enthält die Isophoron enthaltende Zusammensetzung aus mindestens 50 Gew.-% Isophoron, bevorzugt mindestens 80 Gew.-% Isophoron, besonders bevorzugt mindestens 90 Gew.-% Isophoron bezogen auf die Gesamtzusammensetzung.

Es ist erfindungsgemäß bevorzugt, wenn der ausgeleitete Gasstrom durch mehrere, räumlich getrennte Isophoron enthaltende Zusammensetzungen geleitet wird. Hierdurch kann die Rückhaltekapazität gesteigert werden.

Es ist vorteilhaft und bevorzugt, wenn die Isophoron enthaltende Zusammensetzung gekühlt vorliegt. Hierdurch kann - bezogen auf die isophoronhaltige Zusammensetzung - mehr niedermolekulare, organische Verbindung absorbiert werden. In diesem Zusammenhang bevorzugt Temperaturen, gemessen in der Isophoron enthaltenden Zusammensetzung, liegen in einem Bereich von -5 °C bis 50° C, insbesondere von 0 °C bis 30 °C.
Es ist vorteilhaft und daher bevorzugt, wenn das die Isophoron enthaltende Zusammensetzung verlassende Gas oder Gasgemisch der wässrigen Lösung aus Verfahrensschritt A) wieder zugeführt wird.

In Verfahrensschritt D) wird die Trennung der niedermolekularen, organischen Verbindungen, von der Isophoron enthaltenden Zusammensetzung bevorzugt durch eine Druckverränderung und/oder durch eine Temperaturerhöhung (>20 °C bis <200 °C) erfolgen.
So liegen beispielsweise die Siedepunkte bei Normaldruck für Aceton bei 56 °C, für Wasser bei 100 °C, für Isophoron bei 215 °C.
Somit liegen in diesem Zusammenhang für ein System enthaltend Aceton und eine isophoronhaltige Zusammensetzung die bevorzugten Temperaturen in einem Bereich von 20 °C bis 75 °C, insbesondere von 30 °C bis 60 °C, um das Aceton von der Isophoron enthaltenden Zusammensetzung zu trennen, wobei die Temperatur in der Isophoron enthaltenden Zusammensetzung gemessen wird.
In diesem Zusammenhang bevorzugte Drücke liegen in einem Bereich von 0 bis 1,7 bar, besonders bevorzugt bei 0,9 bis 1,1 bar.
Besonders bevorzugt ist in diesem Zusammenhang eine Kombination von Normaldruck (d.h. Umgebungsdruck) und einer Temperatur in Bereich von 30 bis 60 °C

Es ist erfindungsgemäß bevorzugt, wenn mindestens die Verfahrensschritte B) und C) zeitgleich mit der Produktion der niedermolekularen, organischen Verbindung in Verfahrensschritt A) durchgeführt werden, so dass es sich um einen kontinuierlichen *in-situ-*Prozess handelt, bei dem das Produkt während bzw. unmittelbar nach der Entstehung aus der wässrigen Lösung ausgetragen wird.

In den nachfolgend aufgeführten Beispielen wird die vorliegende Erfindung beispielhaft beschrieben, Ein weiterer Gegenstand der vorliegenden Erfindung ist die Verwendung von Isophoron zur Absorption der niedermolekularen, organischen Verbindungen, Aceton, Butanol, Ethanol, insbesondere Aceton aus einem Gas oder Gasgemisch enthaltend die niedermolekulare, organische Verbindung, Aceton, Butanol, Ethanol, insbesondere Aceton.

### Beispiele:

Die Isolierung von organischen Komponenten (Aceton / Ethanol / Butanol) aus einer wässrigen Fermenterlösung wurde untersucht. In allen Beispielen wurde eine im Wesentlichen wässrige Fermenterlösung mit einem organischen Anteil (z. B.: Aceton / Ethanol / Butanol) von max. 15 Gew.% verwendet, um die organischen Bestandteile mittels eines Gasstroms aus dem Fermenter auszustrippen. Die abschließende Aufreinigung und Produktisolierung erfolgte durch destillative Trennung.

### Vergleichsbeispiel 1, nicht erfindungsgemäß:

1 Liter einer wässrigen Lösung mit einem organischen Anteil von 2,0 Gew.% Aceton wurde in einen 2 L-Fermenter gefüllt. Der Fermenter wurde mit 200 rpm gerührt und mit 0,6 Volumen Stripgas pro Volumen Reaktor pro Minute (vvm) begast. Als Strippgas wurde Stickstoff verwendet und die Temperatur der Fermenterlösung wurde auf 34°C eingestellt. An den Fermenter wurden zwei Kühlgefäße nachgeschaltet um das gasförmige Aceton auszukondensieren und somit zu isolieren. Die Auffangbehälter der Kühlgefäße wurden dafür in Trockeneis -78°C gelagert und die Kühler auf 5°C gekühlt.
Anhand dieses Experiments konnte gezeigt werden, dass sich Aceton aus dem Fermenter mittels eines Gases ausstrippen lässt. Nach 5,5 h war nur noch ∼1,0 Gew.% Aceton (∼50% der Ausgangs-Acetonmenge), nach 23 h nur noch ∼0,1 Gew.% Aceton (∼5% der Ausgangs-Acetonmenge) in der wässrigen Fermenterlösung. Die Wiederfindungsrate des ausgestrippten Acetons in den gekühlten Auffangbehältern der Kühlgefäße lag jedoch nur bei <40% nach 23 h, d.h. mehr als 60% des gestrippten Acetons konnten auf diese Weise nicht aufgefangen und isoliert werden.

### Vergleichsbeispiel 2, nicht erfindungsgemäß:

1,4 Liter einer wässrigen Lösung mit einem organischen Anteil von 0,5 Gew.% Aceton wurde in einen 2 L-Fermenter gefüllt. Der Fermenter wurde mit 500 rpm gerührt und mit 0,2 Volumen Stripgas pro Volumen Reaktor pro Minute (vvm) begast. Als Strippgas wurde Stickstoff verwendet und die Temperatur der Fermenterlösung wurde auf 30°C eingestellt. Anstatt der nachgeschalteteten Kühlgefäße wurde der Gasstrom durch sechs in Reihe geschaltete Waschflaschen gefüllt mit dem Lösungsmittel Dimethylsulfoxid (DMSO) geleitet, die an den Fermenter nachgeschaltet waren, um das gasförmige Aceton in DMSO zu absorbieren. Die ersten 5 Waschflaschen waren mit 200 g DMSO gefüllt, die sechste Waschflasche mit 500g DMSO. Aufgrund der geringeren Begasungsrate und der geringeren Ausgangs-Acetonmenge erfolgte die Ausstrippung des Acetons erwartungsgemäß langsamer, nach 23h waren noch ∼0,28 Gew.% Aceton (∼55% der Ausgangs-Acetonmenge), nach 48 h nur noch ∼0,13 Gew.% Aceton (∼27% der Ausgangs-Acetonmenge) in der wässrigen Fermenterlösung. Mit Hilfe der in Reihe geschalteten DMSO-Lösungen wurde die Aceton-Wiederfindungsrate verbessert, diese lag aber immer noch deutlich zu niedrig. Nach 23h lag Wiederfindungsrate des gestrippten Acetons unter 90%, nach 48 h nur noch bei 74%

=> Gasstripping von Aceton aus einer wässrigen Fermenterlösung erfolgreich, aber die Absorbtion des gasförmigen Acetons in dem Lösungsmittel DMSO und somit die Isolation des Acetons war nicht ausreichend.

### Beispiel 1, erfindungsgemäß:

0,3 Liter einer wässrige Lösung mit einem organischen Anteil von 5 Gew.% Aceton, 5 Gew.% Ethanol und 5 Gew.% 1-Butanol wurde in einen 1 L-Fermenter gefüllt. Der Fermenter wurde mit 200 rpm gerührt und mit 0,1 Volumen Stripgas pro Volumen Reaktor pro Minute (vvm) begast. Als Strippgas wurde Stickstoff verwendet und die Temperatur der Fermenterlösung wurde auf 30°C eingestellt. Anstatt der nachgeschalteteten Kühlgefäße wurde der Gasstrom durch vier in Reihe geschaltete Waschflaschen gefüllt mit dem Lösungsmittel Isophoron geleitet, die an den Fermenter nachgeschaltet waren, um das gasförmige Aceton, Ethanol und Butanol in Isophoron zu absorbieren. Die Waschflaschen waren mit jeweils mit 300 g Isophoron gefüllt. Nach 28h waren noch ∼2,50 Gew.% Aceton (∼50% der Ausgangs-Acetonmenge), noch 4,50 Gew.% Ethanol (∼90% der Ausgangs-Ethanolmenge) und noch 4,60 Gew.% 1-Butanol (∼92% der Ausgangs-Butanolmenge) in der wässrigen Fermenterlösung. Nach 68h waren noch ∼0,76 Gew.% Aceton (∼15% der Ausgangs-Acetonmenge), noch 4,03 Gew.% Ethanol (∼80% der Ausgangs-Ethanolmenge) und noch 3,83 Gew.% 1-Butanol (∼76% der Ausgangs-Butanolmenge) in der wässrigen Fermenterlösung.. Mit Hilfe der in Reihe geschalteten Isophoron-Lösungen wurde die Aceton-deutliche Wiederfindungsrate verbessert, und auch die Wiederfindungsraten für Ethanol und 1-Butanol lagen bei deutlich > 90%. Nach 28h lag die Wiederfindungsrate des gestrippten Acetons bei >93%, des gestrippten Ethanols bei >95%, des gestrippten 1-Butanols bei >96%. Nach 68h lag die Wiederfindungsrate des gestrippten Acetons bei >92%, des gestrippten Ethanols bei >93%, des gestrippten 1-Butanols bei >94%. => Gasstripping von Aceton, Ethanol und Butanol aus einer wässrigen Fermenterlösung erfolgreich. Isolierung der organischen Bestandteile (Aceton, Ethanol, 1-Butanol) durch Absorption in dem Lösungsmittel Isophoron erfolgreich, Es konnte somit eine nahezu quantitative Wiederfindungsrate erreicht werden.

### Beispiel 2, erfindungsgemäß:

Um das in Isophoron gelöste Aceton, Ethanol und Butanol als Reinstoff zu erhalten wurde eine solche Mischung destillativ getrennt. Dafür wurden 5 Liter eines Aceton-Ethanol-Butanol-Gemischs (jeweils 5 Gew.%) gelöst in Isophoron angesetzt und destillativ bei Normaldruck getrennt, das Rücklaufverhältnis der Kolonne betrug dabei 8:1. Die Siedepunkte beim Normaldruck liegen für Aceton bei 56°C, für Ethanol bei 78°C, für 1-Butanol bei 118°C und Isophoron bei 215°C. Es wurden dabei sowohl nahezu reine Aceton-, als auch reine Ethanol- und Butanol-Phasen erhalten. Der Reinheitsgehalt der einzelnen isolierten Phasenbetrug dabei bis zu >99% Reinheit.

=> Es ist also möglich das im Fermenter erzeugte Aceton / Ethanol / Butanol *in-situ* aus dem Fermenter durch Gasstrippen auszutreiben, das gasförmige Aceton / Ethanol / Butanol (Absorbat) in flüssigen Isophoron (Absorptionsmittel)zu lösen und zuletzt das Aceton / Ethanol / Butanol durch destillative Trennung zu gewinnen.

## Patentansprüche

1. Verfahren umfassend die Verfahrensschritte
A) Bereitstellen einer wässrigen Lösung umfassend niedermolekulare, organische Verbindungen produzierende Mikroorganismen,
B) Einleiten mindestens eines Gases oder Gasgemisches in die wässrige Lösung,
C) Ausleiten des Gasstromes durch eine Isophoron enthaltende Zusammensetzung und gegebenenfalls
D) Abtrennen der niedermolekularen, organischen Verbindung von der Isophoron enthaltenden Zusammensetzung,
**dadurch gekennzeichnet,**
**dass** die niedermolekulare, organische Verbindung ausgewählt ist aus der Gruppe umfassend, Aceton, Butanol, Ethanol, insbesondere Aceton.

2. Verfahren gemäß Anspruch 1,
**dadurch gekennzeichnet,**
**dass** die Mikroorganismen ausgewählt sind aus Gattungen der Gruppe umfassend *Clostridium, Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Pichia, Candida, Hansenula* und *Saccharomyces.*

3. Verfahren gemäß mindestens einem der vorherigen Ansprüche,
**dadurch gekennzeichnet,**
**dass** das Gas oder Gasgemisch mindestens eines enthält ausgewählt ist aus der Gruppe umfassend Luft, Synthesegas, Stickstoff, Kohlenstoffdioxid, Kohlenstoffmonoxid, Wasserstoff, Sauerstoff und Methan.

4. Verfahren gemäß mindestens einem der vorherigen Ansprüche,
**dadurch gekennzeichnet,**
**dass** die niedermolekularen, organischen Verbindungen Aceton, Ethanol und Butanol sind und *Clostridium acetobutylicum* und *Clostridium beijerinckii* als Mikroorganismen und Gas oder Gasgemische ausgewählt aus der Gruppe umfassend Stickstoff, Luft, Synthesegas, Kohlenstoffdioxid, Kohlenstoffmonoxid, H₂, Methan eingesetzt werden.

5. Verfahren gemäß mindestens einem Ansprüche 1 bis 4,
**dadurch gekennzeichnet,**
**dass** die niedermolekulare, organische Verbindung Aceton ist, und als Mikroorganismen solche eingesetzt werden, die Aceton ausgehend von Acetyl-CoenzymA über
die enzymatische Umsetzung von Acetyl-CoA zu Acetoacetyl-CoA ,
die enzymatische Umsetzung von Acetoacetyl-CoA zu Acetoacetat und CoA, wobei
das CoenzymA nicht auf ein Akzeptor-Molekül übertragen wird und
das Decarboxylieren von Acetoacetat zu Aceton und CO₂ produzieren.

6. Verfahren gemäß mindestens einem Ansprüche 1 bis 4,
**dadurch gekennzeichnet,**
**dass** die niedermolekulare, organische Verbindung Aceton ist, acetogene Mikroorganismen, die Aceton aus mindestens einer Kohlenstoffquelle ausgewählt aus der Gruppe enthaltend Kohlendioxid und Kohlenmonoxid zu bilden vermögen, eingesetzt werden, und das in Verfahrensschritt B) eingesetzte Gas oder Gasgemisch mindestens eines ausgewählt aus der Gruppe umfassend Stickstoff, Synthesegas, Kohlenstoffdioxid, Kohlenstoffmonoxid, H₂ ist.

7. Verfahren gemäß mindestens einem der vorherigen Ansprüche,
**dadurch gekennzeichnet,**
**dass** der ausgeleitete Gasstrom in Verfahrensschritt C) durch mehrere, räumlich getrennte Isophoron enthaltende Zusammensetzungen geleitet wird.

8. Verfahren gemäß mindestens einem der vorherigen Ansprüche,
**dadurch gekennzeichnet,**
**dass** das die Isophoron enthaltende Zusammensetzung verlassende Gas oder Gasgemisch der wässrigen Lösung aus Verfahrensschritt A) wieder zugeführt wird.

9. Verfahren gemäß mindestens einem der vorherigen Ansprüche,
**dadurch gekennzeichnet,**
**dass** in Verfahrensschritt A) die Mikroorganismen die niedermolekularen, organischen Verbindungen unter aeroben Bedingungen produzieren und als Gas oder Gasgemische in Verfahrensschritt B) ein elementaren Sauerstoff enthaltendes Gas oder Gasgemisch eingesetzt wird.

10. Verfahren gemäß mindestens einem der Ansprüche 1 bis 9,
**dadurch gekennzeichnet,**
**dass** in Verfahrensschritt A) die Mikroorganismen die niedermolekularen, organischen Verbindungen unter anaeroben Bedingungen produzieren und in Verfahrensschritt B) ein Gas oder Gasgemisch, welches frei von elementarem Sauerstoff ist, eingesetzt wird.

11. Verwendung von Isophoron zur Absorption von niedermolekularen, organischen Verbindungen ausgewählt aus der Gruppe umfassend, Aceton, Butanol, Ethanol, insbesondere Aceton, aus einem Gas oder Gasgemisch enthaltend die niedermolekulare, organische Verbindung.

## Claims

1. Method comprising the method steps
A) providing an aqueous solution comprising microorganisms producing low-molecular-weight organic compounds,
B) introducing at least one gas or gas mixture into the aqueous solution,
C) channelling the gas stream out through an isophorone-containing composition and optionally
D) separating off the low-molecular-weight organic compound from the isophorone-containing composition,
**characterized in that** the low-molecular-weight organic compound is selected from the group comprising acetone, butanol, ethanol, in particular acetone.

2. Method according to Claim 1, **characterized in that** the microorganisms are selected from genera of the group comprising *Clostridium, Zymomonas, Escherichia, Salmonella, Rhodococcus, Pseudomonas, Bacillus, Lactobacillus, Enterococcus, Acaligenes, Klebsiella, Paenibacillus, Arthrobacter, Corynebacterium, Brevibacterium, Pichia, Candida, Hansenula* and *Saccharomyces.*

3. Method according to at least one of the preceding claims, **characterized in that** the gas or gas mixture contains at least one selected from the group comprising air, synthesis gas, nitrogen, carbon dioxide, carbon monoxide, hydrogen, oxygen and methane.

4. Method according to at least one of the preceding claims, **characterized in that** the low-molecular-weight organic compounds are acetone, ethanol and butanol, and *Clostridium acetobutylicum* and *Clostridium beijerinckii* are used as microorganisms and gas or gas mixtures selected from the group comprising nitrogen, air, synthesis gas, carbon dioxide, carbon monoxide, H₂ and methane.

5. Method according to at least one of Claims 1 to 4, **characterized in that** the low-molecular-weight organic compound is acetone, and the microorganisms used are those which produce acetone starting from acetyl coenzyme A via
the enzymatic reaction of acetyl-CoA to form acetoacetyl-CoA,
the enzymatic reaction of acetoacetyl-CoA to form acetoacetate and CoA, wherein
the coenzyme A is not transferred to an acceptor molecule and
the decarboxylation of acetoacetate to form acetone and CO₂.

6. Method according to at least one of Claims 1 to 4, **characterized in that** the low-molecular-weight organic compound is acetone, acetogenic microorganisms which are able to form acetone from at least one carbon source selected from the group containing carbon dioxide and carbon monoxide are used, and the gas or gas mixture used in method step B) is at least one selected from the group comprising nitrogen, synthesis gas, carbon dioxide, carbon monoxide and H₂.

7. Method according to at least one of the preceding claims, **characterized in that** the gas stream channelled out in method step C) is passed through a plurality of spatially separated isophorone-containing compositions.

8. Method according to at least one of the preceding claims, **characterized in that** the gas or gas mixture leaving the isophorone-containing composition is fed back to the aqueous solution of method step A).

9. Method according to at least one of the preceding claims, **characterized in that**, in method step A), the microorganisms produce the low-molecular-weight organic compounds under aerobic conditions, and as gas or gas mixtures in method step B), a gas or gas mixture containing elemental oxygen is used.

10. Method according to at least one of Claims 1 to 9, **characterized in that**, in method step A), the microorganisms produce the low-molecular-weight organic compounds under anaerobic conditions, and in method step B) a gas or gas mixture which is free of elemental oxygen is used.

11. Use of isophorone for absorption of low-molecular-weight organic compounds selected from the group comprising acetone, butanol, ethanol, in particular acetone, from a gas or gas mixture containing the low-molecular-weight organic compound.

## Revendications

1. Procédé comprenant les étapes de procédé suivantes :
A) la préparation d'une solution aqueuse comprenant des microorganismes produisant des composés organiques de faible poids moléculaire,
B) l'introduction d'au moins un gaz ou mélange de gaz dans la solution aqueuse,
C) l'évacuation du courant gazeux au travers d'une composition contenant de l'isophorone, et éventuellement
D) la séparation du composé organique de faible poids moléculaire de la composition contenant de l'isophorone, **caractérisé en ce que**
le composé organique de faible poids moléculaire est choisi dans le groupe comprenant l'acétone, le butanol, l'éthanol, notamment l'acétone.

2. Procédé selon la revendication 1, **caractérisé en ce que** les microorganismes sont choisis parmi les genres du groupe comprenant *Clostridium, Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Pichia, Candida, Hansenula* et *Saccharomyces.*

3. Procédé selon au moins l'une quelconque des revendications précédentes, **caractérisé en ce que** le gaz ou mélange de gaz contient au moins un gaz choisi dans le groupe comprenant l'air, un gaz de synthèse, l'azote, le dioxyde de carbone, le monoxyde de carbone, l'hydrogène, l'oxygène et le méthane.

4. Procédé selon au moins l'une quelconque des revendications précédentes, **caractérisé en ce que** les composés organiques de faible poids moléculaire sont l'acétone, l'éthanol et le butanol, et *Clostridium acetobutylicum* et *Clostridium beijerinckii* sont utilisés en tant que microorganismes, et un gaz ou mélange de gaz choisi dans le groupe comprenant l'azote, l'air, un gaz de synthèse, le dioxyde de carbone, le monoxyde de carbone, H₂, le méthane est utilisé.

5. Procédé selon au moins l'une quelconque des revendications 1 à 4, **caractérisé en ce que** le composé organique de faible poids moléculaire est l'acétone, et des microorganismes qui produisent de l'acétone à partir d'acétyl-coenzyme A par
la transformation enzymatique d'acétyl-CoA en acétoacétyl-CoA,
la transformation enzymatique d'acétoacétyl-CoA en acétoacétate et CoA,
la coenzyme A n'étant pas transférée sur une molécule accepteur, et
la décarboxylation d'acétoacétate en acétone et CO₂, sont utilisés en tant que microorganismes.

6. Procédé selon au moins l'une quelconque des revendications 1 à 4, **caractérisé en ce que** le composé organique de faible poids moléculaire est l'acétone, des microorganismes acétogènes, qui peuvent former de l'acétone à partir d'au moins une source de carbone choisie dans le groupe contenant le dioxyde de carbone et le monoxyde de carbone, sont utilisés, et le gaz ou mélange de gaz utilisé à l'étape de procédé B) est au moins un gaz choisi dans le groupe comprenant l'azote, un gaz de synthèse, le dioxyde de carbone, le monoxyde de carbone, H₂.

7. Procédé selon au moins l'une quelconque des revendications précédentes, **caractérisé en ce que** le courant gazeux évacué à l'étape de procédé C) est acheminé au travers de plusieurs compositions contenant de l'isophorone séparées dans l'espace.

8. Procédé selon au moins l'une quelconque des revendications précédentes, **caractérisé en ce que** le gaz ou mélange de gaz quittant la composition contenant de l'isophorone est réintroduit dans la solution aqueuse de l'étape de procédé A).

9. Procédé selon au moins l'une quelconque des revendications précédentes, **caractérisé en ce qu'**à l'étape de procédé A), les microorganismes produisent les composés organiques de faible poids moléculaire en conditions aérobies et, en tant que gaz ou mélange de gaz à l'étape de procédé B), un gaz ou mélange de gaz contenant de l'oxygène élémentaire est utilisé.

10. Procédé selon au moins l'une quelconque des revendications 1 à 9, **caractérisé en ce qu'**à l'étape de procédé A), les microorganismes produisent les composés organiques de faible poids moléculaire en conditions anaérobies et, en l'étape de procédé B), un gaz ou mélange de gaz qui est exempt d'oxygène élémentaire est utilisé.

11. Utilisation d'isophorone pour l'absorption de composés organiques de faible poids moléculaire choisis dans le groupe comprenant l'acétone, le butanol, l'éthanol, notamment l'acétone, à partir d'un gaz ou mélange de gaz contenant le composé organique de faible poids moléculaire.
